# EUROPEAN PATENT SPECIFICATION

(11) **EP 4 147 645 B1**
(45) Date of publication and mention of the grant of the patent: **21.02.2024**
(21) Application number: 22192463.2
(22) Date of filing: 26.08.2022
(51) Int. Cl.: A61B 6/03, A61B 6/00, A61G 3/00

(54) **MOBILE COMPUTED TOMOGRAPHY UNIT**
MOBILE COMPUTERTOMOGRAPHIE-EINHEIT
UNITÉ MOBILE DE TOMOGRAPHIE ASSISTÉE PAR ORDINATEUR

(30) Priority: 14.09.2021 IT 202100023609
(43) Date of publication of application: 15.03.2023
(73) Proprietor: Luongo, Jolanda, 25079 Vobarno (BS) (IT)
(72) Inventor: Luongo, Jolanda, 25079 Vobarno (BS) (IT)
(74) Representative: Biesse S.r.l.

(56) References cited:
- CN-A- 111 329 506
- US-A- 4 181 347
- US-A1- 2020 253 574
- TROINA GRÉGORI ET AL: "Computational Modeling and Constructal Design Theory Applied to the Geometric Optimization of Thin Steel Plates with Stiffeners Subjected to Uniform Transverse Load", METALS, vol. 10, no. 2, 4 February 2020 (2020-02-04), page 220, XP055915130, DOI: 10.3390/met10020220
- WALTER SILKE ET AL: "Bringing the Hospital to the Patient: First Treatment of Stroke Patients at the Emergency Site", PLOS ONE, vol. 5, no. 10, 29 October 2010 (2010-10-29), page e13758, XP055915338, DOI: 10.1371/journal.pone.0013758

## Description

### Field of the invention

The present invention concerns a mobile computed tomography CT unit studied and adapted for use on cargo planes, or possible alternative road vehicles, intended to be used in remote areas or in spaces different than those in hospitals.

### Known art

As is known, the computed tomography CT is a second level radiological technique through which it is possible to generate sectional and three-dimensional digital images of the anatomy of a patient, by analyzing the attenuation of an X-ray beam crossing a body section of the patient.

There are areas of our planet that are lacking in hospitals, clinics and health facilities and where it is therefore difficult, if not impossible, to perform a computed tomography CT or other second level exams for the population. The same difficulty can also arise, for example, in war zones or in areas struck by natural disasters, such as earthquakes or floods, which make the existing hospitals unfit for use.

In 2020, with the onset of the Covid-19 pandemic, there was a need to perform screenings and exams outdoors in order to protect the patients inside the hospitals. However, the current health organization is to this day unable to provide a radiological CT investigation service outside of the hospital setting.

The reason behind the difficulties mentioned lies in the fact that the current units used to perform the computed tomography CT, or more simply the *CT units,* are not adapted for being transported. More in detail, a typical CT unit comprises two main components: a circular framework (*gantry*) which defines a portal and a corresponding table mounted slidingly on a base. The tomograph, the tool which generates corresponding X-ray beams, is mounted in the framework. The tomograph is movable along the inner circumference of the framework in both directions, to direct the X-ray beams on a scanning plane, in direction of the mechanical isocenter. The patient lies on the table, which is mounted horizontally; the table is susceptible to controlled movements in both directions, such as to bring the patient at the scanning plane, in the area delimited by the framework, and to extract the patient from the framework once the CT is concluded.

In order to make sure a computed tomography CT can be done with high level of precision, the framework and the table with the respective base must be positioned with maximum precision with respect to each other during the calibration of the mechanical isocenter, during the first installation of the equipment on the floor, since this essential calibration step is provided following the installation. The operators in charge of the works define the *mechanical isocenter* as the proper alignment between the framework and the table; the mechanical isocenter is identified and set up during the initial installation of the equipment and must not be modified, such as not to affect the quality of the diagnostic exam.

Consequently, the possible displacement of the CT unit would cause its temporary disuse. In fact, once the framework and the table with the base should be displaced in a new seat, it would be necessary to identify the mechanical isocenter once more and to perform a new calibration. Such procedure requires a lot of time, quantifiable in more work days, the presence of a specialized technician and, often, also a connection to a mobile data network for the remote connection of the equipment's control software to the manufacturer's servers.

Similarly, also a soft or vibration-transmitting floor would make the CT unit unusable; once the table with the patient have been positioned in the framework, no relative displacements must occur between the table and the framework and possible vibrations must be damped.

By virtue of what has been described, it is clear that transporting a CT unit to quickly perform exams in areas different from a hospital environment, for example on cargo planes, or remote or difficult to access areas, was not a possible path up to today.

US 4,181,347 discloses a transportable computed tomography CT unit, contained within an intermodal (shipping) container equipped as medical clinic, provided with sufficient room, electric generator, etc.. The container is suitable for transport on lorries.

### Summary of the invention

Object of the present invention is to provide a computed tomography CT unit that is portable and immediately operative once the destination has been reached, without having to perform a new calibration (of first installation).

In particular, an object of the present invention is to provide a mobile, air-transportable, CT unit which allows to perform diagnostic exams directly on board, or in realities such as road vehicles, field hospitals, tensile structures, etc.

The present invention thus concerns the mobile CT unit according to claim 1, which has its own flooring, which flooring is constituted by a plate, preferably made of steel, to which the framework and the table are solidly fastened fixedly.

The assembly formed by the plate, by the framework and by the table constitutes a mobile floored CT unit which can be air-transported or transported, without risking losing the calibration, such as no displacements occur on the three axes between the components of the unit itself just described. In other words, the plate that constitutes the flooring ensures the maintenance of the mechanical isocenter of the unit, thus forming a single body.

The plate comprises grooves for engagement with the forks of an outer forklift.

The solution suggested, which actually provides to implement a mobile CT unit thanks to integrated flooring, allows to perform diagnostic exams in areas otherwise not provided by such type of services, for example outdoor areas, war zones or where natural disasters have occurred, etc. Last, but not least, since the mobile CT unit is air-transportable, it is possible to perform exams directly on board of the plane parked in airport rest areas or on alternative landing fields with respect to the airport.

In practice, the mobile CT unit according to the present invention can be loaded aboard military cargo planes, for example Spartan C-27J, or NATO planes or other cargo planes designed for loading the STANDARD NATO 463LHCU-6/E pallets and provided with a special cargo hold and ramp. The CT unit can be supplied by a special generator transported on the plane and which can be connected once the destination has been reached, without needing to assemble and recalibrate the CT unit once more.

Advantageously, the ordinary maintenance of the CT unit can be directly carried out on board of the plane or vehicle.

### Brief list of the figures

Further characteristics and advantages of the invention will become clearer in the review of the following detailed description of its preferred, although not exclusive, embodiments illustrated by way of example and without limitations with the aid of the accompanying drawings, in which:
- figure 1 is a perspective and front view of a CT unit according to the known art;
- figure 2 is a side and elevated view of a framework of a CT unit according to the present invention, shown in its entirety in figure 4;
- figure 3 is a front and elevated view of the framework shown in figure 2;
- figure 4 is a side and elevated view of a CT unit according to the present invention;
- figure 5 is a table which describes the technical characteristics of the CT unit shown in figure 4;
- figure 6 is an isometric and schematic view of a CT unit according to the present invention;
- figure 7 is a plan view of a component of the CT unit according to the present invention;
- figure 8 is a perspective and bottom view of the CT unit shown in figure 6;
- figure 9 is a perspective and side view of a particular of the CT unit shown in figure 6;
- figure 10 is a perspective view of an accessorized component of the CT unit according to the present invention;
- figure 11 is a diagram of the fastening points of the CT unit according to the present invention;
- figure 12 is a table which sets forth the results achieved with simulations relating to the performance of a component of the CT unit according to the present invention;
- figure 13 is a perspective view of a detail of the component shown in figure 7;
- figures 14 and 15 are perspective views which show the results of a finite element analysis on the component shown in figure 7.

### Detailed description of the invention

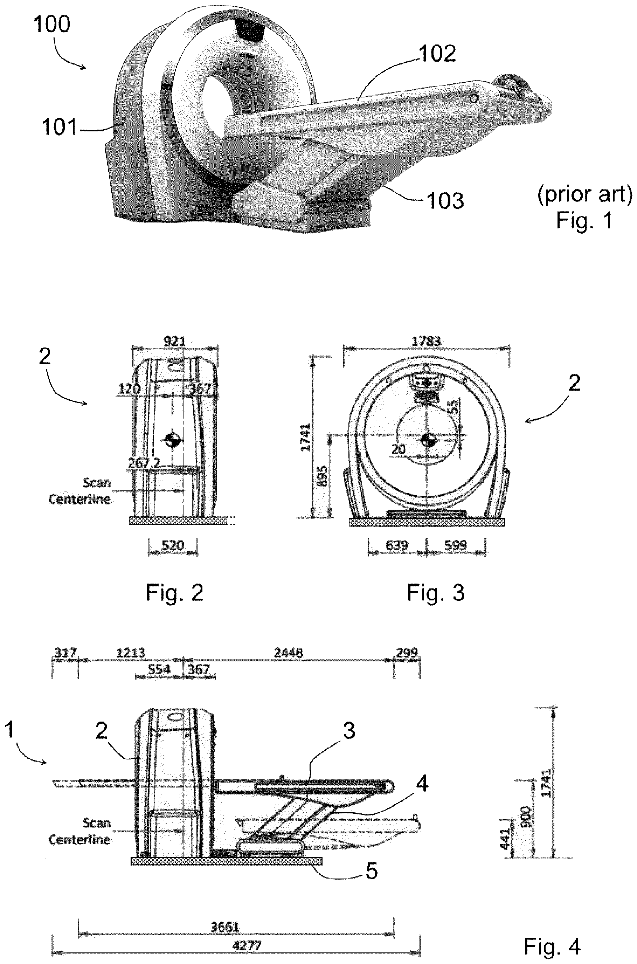

Figure 1 shows a unit 100 for the computed tomography CT made according to the known art, i.e. designed to be fastened to the floor of a hospital environment, for example with plugs, and to stay stationary, not movable.

The unit 100 comprises a framework 101 inside which the tomograph is housed, and a table 102. The table 102 is supported on a base 103 fastened to the floor, whereas the framework 101 extends directly down to the floor to which it is also fastened.

The table 102 is movable in longitudinal direction between an initial position, in which it is fully extracted from the framework, for the ascent and descent of the patient, and a final operative position, in which it is partially inserted into the framework to support the patient at the scanning plane of the tomograph.

Since the purpose is to make a CT unit designed for a stationary use air-transportable, such as to be able to perform diagnoses directly on the plane in the landing area, the Applicant has opted to adopt, among the different possibilities, the CT unit marketed by the company General Electric under the name Revolution Act, since it is a compact unit with respect to most of the others available on the market.

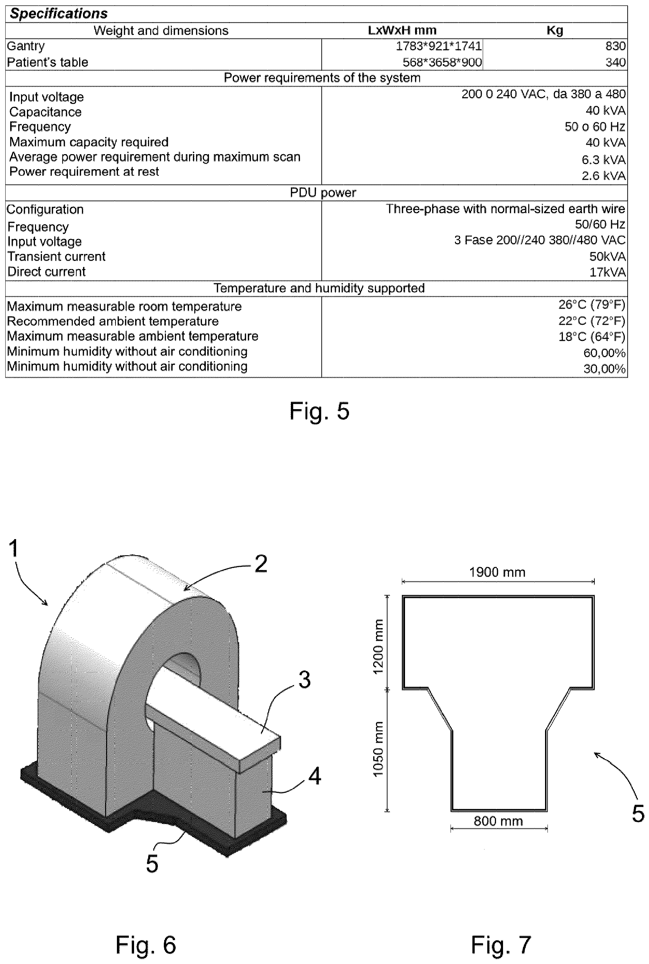

Figure 5 is a table which set forth the main technical specifications of the Revolution Act CT unit adapted to implement the invention. The Revolution Act CT unit has a compact footprint of 8.0 sq.m. and a minimum installation requirement of only 10.1 sq.m.; it is a unit designed for places of very limited size compared to standard-sized CT units that generally require at least 30 sq.m. of useful surface. Moreover, this CT unit is lighter than the average of the other CT units, weighing about 1525 kg. The absorbed power is also lower than the average, generally being less than 40 kW, about 40% less than the average.

Clearly, the present invention is not limited to this model 100 of CT unit, which model must be understood as a suggestion.

Figures 2 and 3 schematically show the framework 2 of the CT unit selected as a starting point to make the mobile air-transportable unit 1 according to the present invention. In particular, for illustration purposes only, the dimensions of the framework 2 are denoted in millimeters. As can be noted, the imprint on the ground has a footprint equal to 520 x 1238 mm.

Figure 4 is a schematic, side and elevation view of a floored unit 1 according to the present invention, i.e. the same is provided with a plate 5 acting as a floor but which allows it to be transported on a cargo plane.

The unit 1 can thus be made by installing a compact CT unit on a plate 5, such as a relative displacement cannot occur between the base 4 of the table 3 and the framework 2 and, thus, such as the assembly of components just described is monolithic and the unit 1 is transportable without losing the initial calibration.

The unit 1 according to the present invention can thus be defined as a mobile floored CT unit.

The plate 5 was conceived to allow to be transported on cargo planes, since it is not possible to use the traditional pallets of the planes themselves. For example, the pallet NATO 463L, which is the standard of air transport on military cargo planes, consists of a rectangular aluminum structure with a soft wood core (balsa). Clearly, this structure is not sufficiently resistant for supporting a CT unit without bending, being subject to twisting and, in general, without deforming, despite its nominal load is equal to 3400 kg, corresponding to 57.9% of its maximum load, which is equal to 4500 Kg. Moreover, a NATO pallet would not ensure the tightness of the fastening screws of the CT unit due to the soft nature of balsa wood.

Clearly, the plate 5, which constitutes the floor of the floored and mobile CT unit 1, is also transportable on other means of transport, for example trucks, since they are subject to less stress than a plane.

Figure 6 is a perspective view of the mobile and floored CT unit 1 according to the present invention, only schematic for simplicity. The framework 2 and the table 3 with the base 4 are fastened to the plate 5 and constitute a rigid assembly.

Figure 7 is a plan view of the plate 5: purely by way of example, the footprints are denoted in millimeters and are slightly greater than the footprints of the framework 2 and the table 3. Preferably, the plate 5 is made of stainless steel, for example of the 1.4301 type.

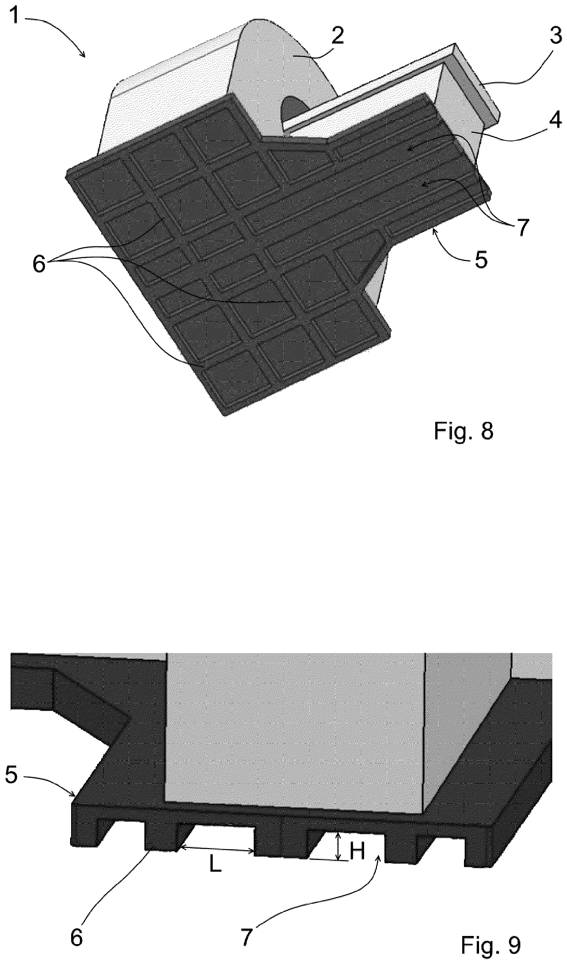

Figure 8 is a perspective bottom view of the CT unit 1 schematically shown in figure 6. As can be noted, the plate 5 has a plurality of stiffening ribs 6 which have the function of preventing the plate 6 from twisting. Preferably, the ribs have a width L equal to 150 mm and a height H equal to 75 mm.

As shown in the drawing, the ribs 6 define two guides or longitudinal grooves 7 intended to house the forks of a forklift, through which forklift the unit 1 can be moved, as an alternative to using a crane or a mechanical arm. The forklift is in fact the means generally used to load and unload goods on/off cargo planes.

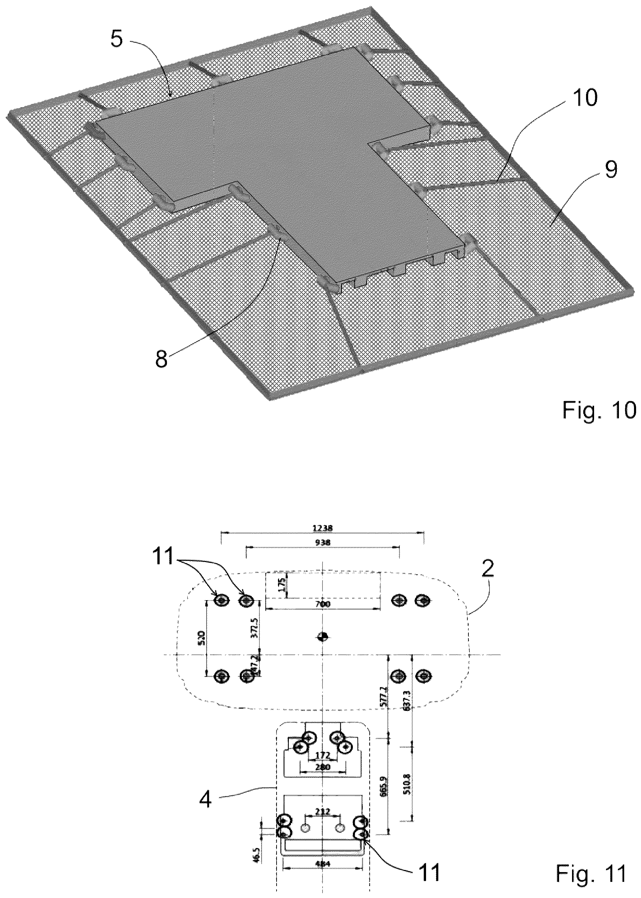

Figure 10 is a perspective view of the plate 5 equipped with fifteen handles 8, six per side and three back ones, which handles allow the anchoring of the plate 5 and, thus, of the CT unit 1 to the pallets or floor 9 of the vehicle used for the transport. In the example shown in the figures, the plate 5 is anchored to the pallet 9 by means of straps 10, e.g. ratchet straps tensioned between the handles 8 and special rings present on the edges of the pallet 9. In the example shown, the handles 8 have a diameter of 50 mm and are welded to the plate 5, such as to extend cantileverly from the edge with an inclination of about 45°, thus staying spaced by about 100 mm. Preferably, the welding is carried out by using steel 1.4301 according to the AISI standard, for example by preferring a full penetration joint. Preferably, the handles are 100 mm wide and 80 mm high.

Since the NATO pallets are slidingly insertable into corresponding guides of the cargo hold of military cargo planes provided with roller conveyors and adjustable end stops, the configuration described makes the CT unit 1 particularly suitable to be transported on planes of this type, such as the Spartan C27J or NATO planes or other cargo planes designed for loading the STANDARD NATO 463LHCU-6/E pallets.

Considering the mass of the CT unit 1, it was calculated that this system allows to effectively support the CT unit 1 in the cargo hold of a military cargo plane, for the normal gravitational accelerations and stress to which these planes are subjected.

Figure 11 shows the scheme for fastening the framework 2 and the base 4 of the table 3 to the plate 5. Sixteen fastening bolts, eight intended to lock the framework 2 on the plate 5 and eight intended to lock the base 4 of the table 3 on the plate 5, are denoted by the number of reference 11. By way of example, the dimensions, in millimeters, for the execution of the holes through the plate 5 are shown. For example, bolts adapted for the purpose have the following characteristics:
- diameter = M12 (x1.75);
- quality = 10.9;
- tightening torque = 80 Nm;
- length of the threaded portion = 12 mm.

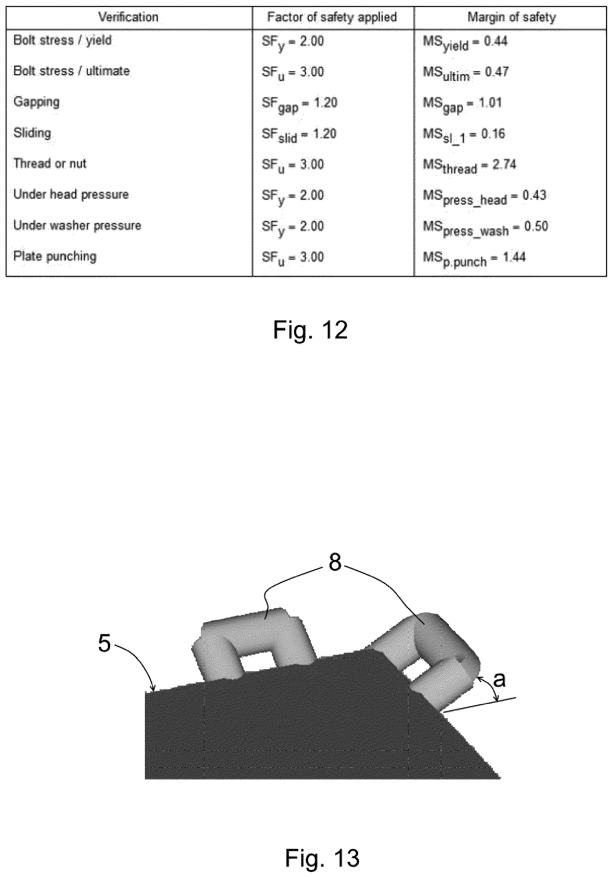

It was calculated that these bolts effectively support accelerations equal to 1.5g on the horizontal plane (x, y) and accelerations equal to 4g on the vertical plane (z), which are the nominal accelerations of a Spartan C-27J plane. The table in figure 12 sets forth the safety margins calculated for the accelerations just described.

Figure 13 shows, in perspective, an enlargement of the plate 5 on which two handles 8 are visible. As mentioned above, the angle a of inclination with respect to the plane of the upper surface of the plate 5 is equal to 45°.

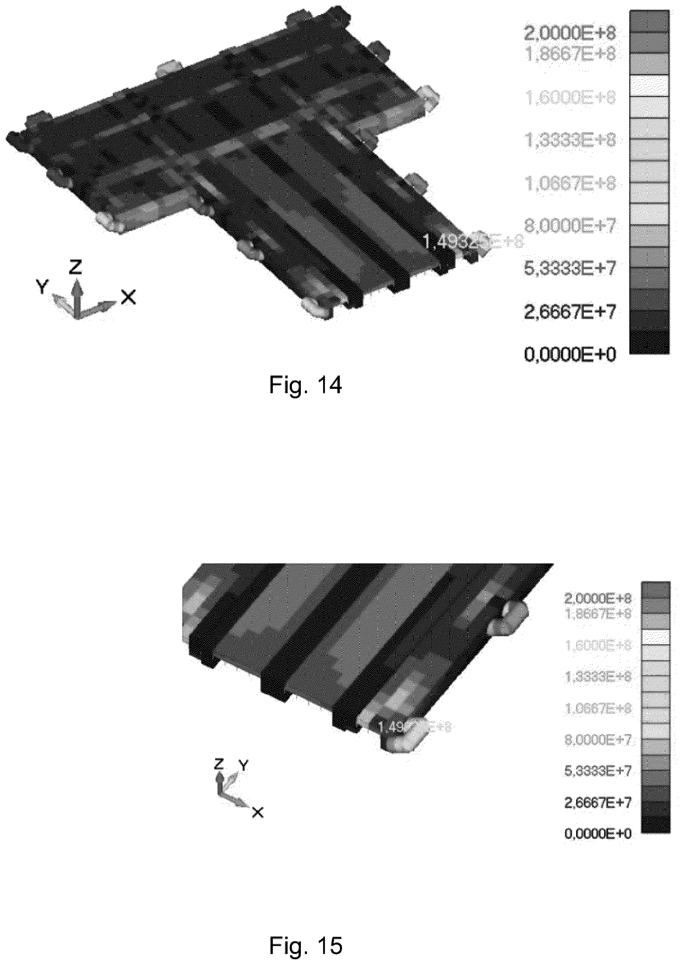

Figures 14 and 15 are colored figures which show the result of a finite element analysis aimed at identifying the points of greater stress of the plates 5 provided with handles 8. As can be noted, the greatest stresses occur on the handles 8 placed at the corners of the plate 5. The maximum stress simulated is equal to 150 Mpa, lower than the breaking load of steel 1.4301, which is equal to 200 Mpa.

Thanks to the details described above, the CT unit 1 is a mobile unit, air-transportable or transportable on road vehicles, which unit can be immediately used once arrived at destination without requiring recalibration, thanks to the fact that the framework 2 and the base 4 with the table 3 stay fixed in the positions assumed during the first installation.

This way, it is possible to transport the CT unit 1 anywhere where there is a need to perform the diagnostic exam outside of hospital facilities, for example outdoors or directly on board of the plane.

The compact dimensions of the CT unit 1 allow to perform the ordinary maintenance directly on board of the cargo plane, i.e. without needed to transport the CT unit 1 to an indoor environment.

Preferably, the CT unit 1 is also provided with a special auxiliary electricity-generating set, for example of diesel type, which can be positioned at a given distance, at least 15 meters, from the CT unit 1 and connected thereto to provide the necessary power, especially in areas with high temperatures.

Similarly, the CT unit 1 is preferably further provided with a cooling unit configured to maintain the temperature of the CT unit 1 itself around 22°C. The cooling unit can be selected depending on the destination to be reached, i.e. the climate where the unit 1 will be operating. Shielding panels can also be transported on board of the vehicle to protect the health care personnel from ionizing radiation.

## Claims

1. A mobile computed tomography CT unit (1), comprising a framework, (2) provided with one or more tomographs, a table (3), intended to support a lying down patient and movable with respect to the framework (2), and a plate (5) to which the framework (2) and the table (3) are fastened, wherein the framework (2), the table (3) and the plate (5) form a single portable rigid structure as a mobile computed tomography CT unit, in a monolithic way, **characterized in that** the plate (5) comprises grooves (7) for engagement with the forks of an outer forklift.

2. Mobile computed tomography CT unit (1) according to claim 1, wherein the plate (5) is made of metal, preferably made of steel.

3. Mobile computed tomography CT unit (1) according to claim 1 or claim 2, wherein the plate (5) comprises stiffening ribs (6).

4. Mobile computed tomography CT unit (1) according to any one of the preceding claims, wherein the table comprises a leaning base (4), the framework (2) and the leaning base (4) are fastened to the plate (5) by means of bolts (11) during the first installation, just before the initial calibration of the mechanical isocenter of the mobile CT unit.

5. Mobile computed tomography CT unit (1) according to any one of the preceding claims, wherein the plate (5) has plan dimensions slightly greater than the footprint of the framework (2) and the base (4) of the table (3).

6. Mobile computed tomography CT unit (1) according to any one of the preceding claims, wherein the plate (5) is provided with a plurality of perimeter handles (8) which allow to fasten it with straps to outer structures.

7. Mobile computed tomography CT unit (1) according to claim 6, wherein the handles (8) are welded to the plate (5) and define, with the upper level of the plate (5), an angle (a) of about 45°.

8. Mobile computed tomography CT unit (1) according to any one of preceding claims 6-7, wherein the plate (5) and the handles (8) are made of stainless steel 1.4301.

9. Use of the mobile computed tomography CT unit (1) according to any one of the preceding claims, transported on cargo planes and road vehicles or, to perform examinations in open or remote areas, possibly directly on board of the road vehicle or plane, or in field tensile structures.

## Patentansprüche

1. Mobile Computertomographie-CT-Einheit (1), umfassend einen Rahmen (2), der mit einem oder mehreren Tomographen versehen ist, einen Tisch (3), der zum Tragen eines liegenden Patienten bestimmt ist und gegenüber dem Rahmen (2) beweglich ist, sowie eine Platte (5), an der der Rahmen (2) und der Tisch (3) befestigt sind, wobei der Rahmen (2), der Tisch (3) und die Platte (5) eine einzige tragbare starre Struktur als mobile Computertomographie-CT-Einheit in monolithischer Weise bilden, **dadurch gekennzeichnet, dass** die Platte (5) Nuten (7) zum Eingriff mit den Zinken eines äußeren Gabelstaplers aufweist.

2. Mobile Computertomographie-CT-Einheit (1) nach Anspruch 1, wobei die Platte (5) aus Metall, vorzugsweise aus Stahl, besteht.

3. Mobile Computertomographie-CT-Einheit (1) nach Anspruch 1 oder Anspruch 2, wobei die Platte (5) Verstärkungsrippen (6) umfasst.

4. Mobile Computertomographie-CT-Einheit (1) nach einem der vorhergehenden Ansprüche, wobei der Tisch eine Auflagebasis (4) aufweist und der Rahmen (2) und die Auflagebasis (4) mittels Bolzen (11) an der Platte (5) bei der ersten Installation, unmittelbar vor der erstmaligen Kalibrierung des mechanischen Isozentrums der mobilen CT-Einheit, befestigt sind.

5. Mobile Computertomographie-CT-Einheit (1) nach einem der vorhergehenden Ansprüche, wobei die Platte (5) Grundrissabmessungen aufweist, die geringfügig größer sind als die Grundfläche des Rahmens (2) und der Basis (4) des Tisches (3).

6. Mobile Computertomographie-CT-Einheit (1) nach einem der vorhergehenden Ansprüche, wobei die Platte (5) mit einer Vielzahl von Umfangsgriffen (8) versehen ist, die ihre Befestigung mit Gurten an äußeren Strukturen ermöglichen.

7. Mobile Computertomographie-CT-Einheit (1) nach Anspruch 6, wobei die Griffe (8) an der Platte (5) geschweißt sind und mit der oberen Ebene der Platte (5) einen Winkel (a) von etwa 45° definieren.

8. Mobile Computertomographie-CT-Einheit (1) nach einem der vorhergehenden Ansprüche 6-7, wobei die Platte (5) und die Griffe (8) aus Edelstahl 1.4301 bestehen.

9. Verwendung der mobilen Computertomographie-CT-Einheit (1) nach einem der vorhergehenden Ansprüche, transportiert auf Frachtflugzeugen und Straßenfahrzeugen oder zur Durchführung von Untersuchungen in offenen oder abgelegenen Gebieten, möglicherweise direkt an Bord des Straßenfahrzeugs oder Flugzeugs oder in Feld-Zeltstrukturen.

## Revendications

1. Une unité mobile de tomodensitométrie CT (1), comprenant un bâti (2) pourvu d'un ou plusieurs tomographes, une table (3) destinée à supporter un patient allongé et mobile par rapport au bâti (2), et une plaque (5) à laquelle le bâti (2) et la table (3) sont fixés, dans laquelle le bâti (2), la table (3) et la plaque (5) forment une seule structure rigide portable comme unité mobile de tomodensitométrie CT, de manière monolithique, **caractérisée en ce que** la plaque (5) comprend des rainures (7) pour s'engager avec les fourches d'un chariot élévateur externe.

2. Unité mobile de tomodensitométrie CT (1) selon la revendication 1, dans laquelle la plaque (5) est réalisée en métal, de préférence en acier.

3. Unité mobile de tomodensitométrie CT (1) selon la revendication 1 ou 2, dans laquelle la plaque (5) comprend des nervures de raidissement (6).

4. Unité mobile de tomodensitométrie CT (1) selon l'une quelconque des revendications précédentes, dans laquelle la table comprend une base de soutien (4), le bâti (2) et la base de soutien (4) sont fixés à la plaque (5) au moyen de boulons (11) lors de la première installation, juste avant le calibrage initial de l'isocentre mécanique de l'unité mobile CT.

5. Unité mobile de tomodensitométrie CT (1) selon l'une quelconque des revendications précédentes, dans laquelle la plaque (5) présente des dimensions en plan légèrement supérieures à l'empreinte du bâti (2) et de la base (4) de la table (3).

6. Unité mobile de tomodensitométrie CT (1) selon l'une quelconque des revendications précédentes, dans laquelle la plaque (5) est pourvue d'une pluralité de poignées périphériques (8) qui permettent de la fixer avec des sangles aux structures externes.

7. Unité mobile de tomodensitométrie CT (1) selon la revendication 6, dans laquelle les poignées (8) sont soudées à la plaque (5) et définissent, avec le niveau supérieur de la plaque (5), un angle (a) d'environ 45°.

8. Unité mobile de tomodensitométrie CT (1) selon l'une quelconque des revendications 6-7, dans laquelle la plaque (5) et les poignées (8) sont réalisées en acier inoxydable 1.4301.

9. Utilisation de l'unité mobile de tomodensitométrie CT (1) selon l'une quelconque des revendications précédentes, transportée à bord d'avions-cargos et de véhicules routiers ou, pour effectuer des examens dans des zones en plein air ou lointaines, éventuellement directement à bord du véhicule routier ou avion, ou dans des tenso-structures sur le terrain.
